# EUROPÄISCHE PATENTSCHRIFT

(11) **EP 1 395 334 B1**
(45) Veröffentlichungstag und Bekanntmachung des Hinweises auf die Patenterteilung: **04.01.2006**
(21) Anmeldenummer: 02771585.3
(22) Anmeldetag: 23.05.2002
(51) Int. Cl.: A61N 1/32

(54) **EINRICHTUNG ZUM EINBRINGEN VON STOFFEN**
DEVICE FOR APPLYING SUBSTANCES
DISPOSITIF D'INTRODUCTION DE SUBSTANCES

(30) Priorität: 25.05.2001 AT 8332001
(43) Veröffentlichungstag der Anmeldung: 10.03.2004
(73) Patentinhaber: Nova Technical Research GmbH, 1040 Wien (AT)
(72) Erfinder: WALLA, Thomas, A-1190 Wien (AT)
(74) Vertreter: Krause, Peter
(86) Internationale Anmeldenummer: PCT/AT2002/000158
(87) Internationale Veröffentlichungsnummer: WO 2002/094369

(56) Entgegenhaltungen:
- WO-A-01/12260
- WO-A-01/13989
- US-A- 5 169 384
- US-A- 5 807 306
- US-A- 6 029 090
- US-A- 6 030 375

## Beschreibung

Die Erfindung betrifft eine Einrichtung zum Einbringen von Stoffen, wie Medikamente, die in flüssiger, salbenförmiger oder gelförmiger Konsistenz vorliegen, durch die Haut, insbesondere durch Iontophorese, wobei die Resorption des Stoffes durch ein elektrisches Feld, das durch einen definierten Gleichstrom zwischen zwei Elektroden erzeugbar ist, erfolgt,

Im Naturheilkundelexikon, 2000 MZ-Verlag, ist lontophorese derart definiert, daß dies die Anwendung des Gleichstromes zur gesteigerten Resorption auf die Haut gebrachter Medikamente ist. Die Medikamente müssen ionisierbar sein. Die Elektrode wird direkt auf die mit dem Medikament benetzte Haut gebracht. Je nach Angabe wird entweder die Anode oder die Kathode mit der Haut in Kontakt sein. Die Menge des eingeschleusten Medikamentes ist abhängig von der Stromstärke, der Behandlungsdauer und der Größe der Elektrodenfläche. Bei diesem Verfahren macht man sich die elektrische Ladung von Ionen oder Molekülen zunutze. Da sich entgegengesetzte Ladungen anziehen, wandern negative Ladungen zur positiven Elektrode und positive Ladungen zur negativen Elektrode, wenn eine Gleichspannung anliegt.

Anwendung findet die lontophorese beispielsweise bei Muskel- oder Skeletterkrankungen, durch Einbringen von Salben in den Körper.

Die Iontophorese mit Leitungswasser hat sich aber auch als Behandlungsform bei übermäßigen Schwitzen an Händen, Füßen und in der Achselhöhle bewährt.

Es sind aber auch Leiden, speziell bei Frauen, bekannt, die vorwiegend über operative Eingriffe gelindert bzw. geheilt werden. So ist die Inkontinenz ein weitverbreitetes Leiden, das das Lebensgefühl drastisch beeinträchtigt. Der Grund für diese Symptomatik liegt in einer Schwächung der Beckenbodenmuskulatur, die unter anderem dazu führt, daß der Winkel zwischen Harnblase und Harnleiter verändert und die Schließmuskulatur - bei der durch den geschwächten Beckenboden durchhängenden Harnröhre - nur noch eingeschränkt funktioniert. Die Ursachen der Schwächung der Beckenbodenmuskulatur kann einerseits durch Geburt eines Kindes, anlage- oder altersbedingt sein.

Es sind nun Bestrebungen im Gange, durch konservative, nichtoperative Behandlungsmethoden Heilung zu erzielen. Dies im Gegensatz zu operativen Eingriffen. Im Zuge dieser nichtoperativen Behandlungsformen werden mechanisches Muskeltraining der Beckenbodenmuskulatur, Elektrostimulation und medikamentöse Therapieansätze zum Einsatz kommen.

Aufgabe der Erfindung ist es, eine Einrichtung der eingangs genannten Art zu schaffen, die mit einer nichtoperativen Behandlung, die Einbringung entsprechend geeigneter Medikamente zur Linderung oder Heilung von Leiden gezielt im Bereich für die Ursachen der Leiden ermöglicht.

Die Aufgabe wird durch die Erfindung gelöst.

Die erfindungsgemäße Einrichtung ist dadurch gekennzeichnet, daß ein in Körperöffnungen einführbarer, kapselartiger, hermetisch dichter Behälter vorgesehen ist, der mindestens zwei Elektroden zum Aufbau des elektrischen Gleichfeldes an seiner Außenseite aufweist und daß über den Elektroden eine Vorrichtung zur Aufnahme des einzubringenden Stoffes vorgesehen ist. Mit der Erfindung ist es erstmals möglich, je nach Ursache der Leiden und der Örtlichkeit entsprechende Medikamente, insbesondere lontophoresepräparate, mittels lontophorese in Körperöffnungen gezielt einzubringen. Durch das Zusammenwirken vom lontophoresegerät und lontophoresepräparat kann eine gezielte, optimale Behandlung durchgeführt werden.

Dies wird dadurch unterstützt, daß die Anordnung der Elektroden auf entsprechender mathematischer Simulation der Verhältnisse in der gewählten Körperöffnung basiert. Eine gleichmäßige Durchflutung des zu behandelnden Bereiches und damit auch eine optimierte Einbringung der Präparate, auch in den Tiefen der Körperöffnungen, ist damit gewährleistet.

Ein weiterer Vorteil der Erfindung ist darin zu sehen, daß die Behandlung ohne Unterbrechung des Tagesablaufes durchgeführt werden kann, da die erfindungsgemäße Einrichtung in der Körperöffnung getragen wird und keinerlei externe Einrichtungen während der Behandlung benötigt.

Gemäß einem besonderen Merkmal der Erfindung ist der Behälter in Kontakt mit einer Schleimhaut und/oder Haut in einer Körperöffnung, insbesondere im Urogenital- und/oder Vaginal- und/oder Analtrakt und /oder in der Mund- und/oder in der Ohren- und/oder in der Nasenhöhle, angeordnet. Bedingt durch die Wirkung des elektrischen Feldes und der Empfindlichkeit der Haut bzw. der Schleimhäute ist die Möglichkeit einer erfolgversprechenden Behandlung gegeben, die einen operativen Eingriff eventuell vermeiden läßt.

Gemäß der Erfindung steht eine programmierbare Stromquelle mit den Elektroden in Verbindung. Dadurch können die lontophoreseparameter, wie Stromstärke, Pulsverhältnis und Applikationsdauer entsprechend dem Behandlungsplan gewählt werden.

Gemäß einer weiteren Ausgestaltung der Erfindung ist durch eine integrierte, elektrisch heizbare Wärmefläche die Oberfläche des kapselartigen Behälters definiert erwärmbar. Durch die definierte Erwärmung kann eine steigende Medikamentenwirkung erreicht werden.

Nach einer besonderen Ausführungsvariante der Erfindung erfolgt die Energieversorgung in Form eines in den hermetisch dichten Behälter integrierten wiederaufladbaren Akkumulators. Der Vorteil dieser Ausführung liegt auf der Hand. Der wiederaufladbare Akkumulator wird in den Behandlungspausen, wenn dieser also nicht in einer Körperöffnung angeordnet ist, über ein externes Ladegerät mit Energie versorgt, wodurch ein sicherer Umgang mit der erfindungsgemäßen Einrichtung gewährleistet ist.

Gemäß einer weiteren Ausführungsvariante erfolgt die Energieversorgung in induktiver Form von einem externen System. Von Vorteil bei dieser Ausführung ist, daß die erfindungsgemäße Einrichtung gegebenenfalls die Körperöffnung nicht verlassen muß. Auf Grund des heutigen Standards der Technik ist eine derartige Energieversorgung durchaus denkbar.

Nach einer besonderen Weiterbildung der Erfindung sind über eine kontaktlose oder kontaktbehaftete Schnittstelle die Parameter der programmierbaren Stromquelle und die Erwärmung einer integrierten Wärmefläche einstellbar. Damit kann sichergestellt werden, daß nur eine befugte Person, wie beispielsweise der Arzt, die Parameter an die individuellen Bedürfnisse der Patientin bzw. des Patienten oder des Behandlungsplanes anpassen bzw. verändern kann.

Gemäß einer weiteren Ausgestaltung der Erfindung ist der Sollwert und die Form des Stromes über eine kontaktlose oder kontaktbehaftete Schnittstelle entsprechend den Einstellungen in digitaler Form vorgebbar. Dadurch ist eine überaus einfache Programmierung zu erreichen.

Nach einer Weiterbildung der Erfindung vergleicht die Stromquelle den eingestellten Sollwert mit dem gemessenen IST-Wert und regelt die Differenz aus.
Dadurch können die Behandlungsparameter in einfachster Art und Weise sichergestellt werden.

Gemäß einer besonderen Ausgestaltung der Erfindung ist durch entsprechende Schutzmaßnahmen im Ausgang der Stromquelle ein Übersteigen der Leerlaufspannung über einen Grenzwert, beispielsweise über 80 V, vermeidbar.
Damit kann ein sicherer Betrieb gewährleistet werden und es können auch die verschiedensten Normen erfüllt werden.

Nach einer vorteilhaften Weiterbildung der Erfindung ist die Dauer des Stromflusses über eine kontaktlose oder kontaktbehaftete Schnittstelle in digitaler Form programmierbar. Dadurch können in einfachster Weise Behandlungsparameter eingestellt werden.

Gemäß einer weiteren Ausgestaltung der Erfindung steigt der Strom am Beginn der programmierten Zeit rampenförmig bis zum Erreichen des programmierten Sollwertes an und sinkt am Ende rampenförmig auf Null. Dadurch können Irritationen der Haut bzw. der Schleimhaut vermieden werden.

Nach einer weiteren vorteilhaften Ausbildung der Erfindung ist im Falle der externen Unterbrechung der eingestellten Zeit die auf die programmierte Gesamtzeit verbleibende Restzeit abspeicherbar und im Falle des Wiedereinschaltens ist die Restzeit zur Energieabgabe verfügbar. Damit kann in komfortabelster Weise die Behandlungszeit optimal eingehalten werden.

Gemäß einer besonderen Ausgestaltung der Erfindung ist die Stromquelle über einen Berührungsschalter ein- und ausschaltbar. Derartige Schalter sind einfach zu betätigen und sind für das Gesamtkonzept der erfindungsgemäßen Einrichtung als kapselartiger, hermetisch dichter Behälter äußerst geeignet.

Nach einer Weiterbildung der Erfindung ist eine mittels der kontaktlosen oder kontaktbehafteten Schnittstelle programmierbare Regelung für Temperatur und Zeit zur Ansteuerung der in die Wand des kapselartigen Behälters integrierten elektrischen Wärmefläche realisierbar. Auch damit können verschiedenste Parameter der Behandlung in einfachster Form verändert werden, um ein gutes Heilungsergebnis zu erreichen.

Gemäß einem besonderen Merkmal der Erfindung sind die Vorrichtung zur Aufnahme des einzubringenden Stoffes, insbesondere eines Medikamentes, zum Beispiel offenporige Schaumstoffstripps, die mit den anzuwendenden Stoff definiert getränkt und/oder versehen sind. Diese Ausführung trägt zu einer optimalen Einbringung der Medikamente bei. Auch ist dieser Verwendung derartiger Schaumstoffstripps ein äußerst wirtschaftlicher Aspekt abzugewinnen.

Nach einer weiteren Ausführung der Erfindung sind als Stoffträger gelartige Formstücke vorgesehen. Auch derartige Formstücke können in vorteilhafter Art und Weise eingesetzt werden.

Gemäß einer anderen Ausführung der Erfindung sind als Stoffträger Vliese od. dgl. vorgesehen. Auch derartige Stoffträger sind zur Verwendung geeignet.

Nach einem besonderen Merkmal der Erfindung ist auf der Außenseite des hermetisch dichten Behälters mindestens ein Drucksensor zur Messung des Gewebetonus angebracht. Mit einem derartigen Drucksensor kann indirekt der Behandlungserfolg gemessen werden. Die Wirkung der Medikamente wird quasi auf dem Gewebetonus meßtechnisch überwacht.

Gemäß einer Weiterbildung der Erfindung sind die Messwerte des Drucksensors in einem nichtflüchtigen Speicher aufzeichenbar sind. Damit kann die Wirkung der Medikamente über einen gewünschten Zeitraum beobachtet werden.

Nach einer weiteren Ausgestaltung der Erfindung sind die aufgezeichneten Druckmesswerte über eine kontaktlose oder kontaktbehaftete Schnittstelle auslesbar. Dadurch kann der Heilungserfolg reproduzierbar und nachvollzogen werden.

Die Erfindung wird im nachfolgenden anhand eines in der Zeichnung dargestellten Ausführungsbeispieles näher erläutert.

Es zeigt:
- Fig. 1: eine schematische Darstellung der Einrichtung,
- Fig. 2: eine Detailansicht einer Elektrode und
- Fig. 3: einen Prinzipschaltplan der Einrichtung.

Einführend sei festgehalten, daß gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich, usw. auf die unmittelbar beschriebene sowie dargestellte Figur bezogen und sind bei einer Lageänderung sinngemäß auf die neue Lage zu übertragen. Weiters können auch Einzelmerkmale oder Merkmalskombinationen aus dem gezeigten und beschriebenen Ausführungsbeispiel für sich eigenständige, erfinderische oder erfindungsgemäße Lösungen darstellen.

Die den eigenständigen erfinderischen Lösungen zugrundeliegende Aufgabe kann der Beschreibung entnommen werden.

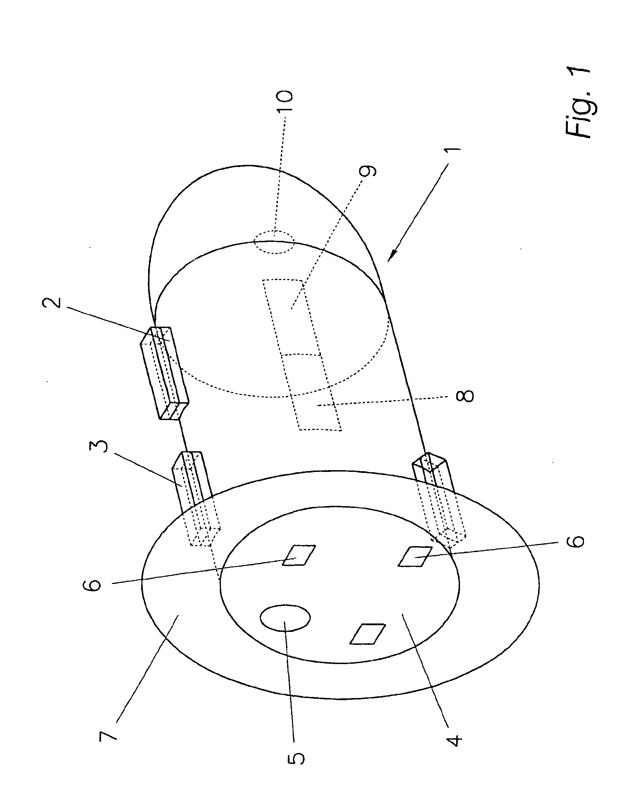

Gemäß der Fig. 1 ist eine Einrichtung, die als Vaginal-lontophoresegerät verwendbar ist, aufgezeigt. Dieses Vaginal-lontophoresegerät wird in den Vaginaltrakt eingeführt und dient zum Einbringen von Stoffen, insbesondere von Medikamenten, durch die Schleimhaut. Das Vaginal-lontophoresegerät besteht aus einem zylindrischen, kapselartigen, hermetisch dichten Behälter 1, der an seiner Außenseite mindestens zwei Elektroden 2 aufweist. Über den Elektroden 2 ist eine Vorrichtung 3 zur Aufnahme des einzubringenden Medikamentes vorgesehen. An einem Ende des zylindrischen Behälters 1 ist dieser kapselartig mit einer Halbkugel verschlossen. Das andere Ende des Behälters 1 ist mit einer Abdeckplatte 4 hermetisch verschlossen, wobei in diese Abdeckplatte 4 ein Berührungsschalter 5 und Schnittstellen-Kontakte 6, beispielsweise für die Übertragung von Daten, integriert sind. Die Abdeckplatte 4 ist in diesem Fall mit einem Verdrehungsschutz 7, der auch als Einführhilfe dienen kann, ausgestattet.

Das Vaginal-Iontophoresegerät weist im Inneren des Behälters 1 ein elektronisches System 8, das beispielsweise von einem wiederaufladbarem, integriertem Akkumulator 9 versorgt wird, auf, wobei voreinstellbare Konstantströme beispielsweise im Bereich von 0,1 mA bis zu 10mA Gleichstrom abgeben werden können. Je nach verwendetem Medikament kann das Pulsverhältnis beispielsweise im Bereich von 1 zu 15 bis zu Konstantstrom voreingestellt werden. Natürlich könnte die Energieversorgung auch in induktiver Form von einem externen System erfolgen.

Die Anordnung der Elektroden 2 ist, basierend auf entsprechender mathematischer Simulation der Verhältnisse im Urogenitaltrakt der Frau, dergestalt, daß eine gleichmäßige Durchflutung des Beckenbodenbereiches und damit eine optimierte Einbringung der Präparate, auch in die Tiefen der Beckenbodenmuskulatur, gewährleistet ist.

Das anzuwendende Medikament bzw. Präparat wird in der Vorrichtung 3 in Form von Medikamentenstripps, das sind offenporige Schaumstoffstreifen, die mit dem jeweiligen Präparat in entsprechender Konzentration getränkt sind, in physikalischem Kontakt mit den Elektroden 2 gebracht. Dadurch ist es möglich, durch Austausch der einmalig anzuwendenden Medikamentenstripps einfachst verschiedene Präparatkonzentrationen und Typen im Rahmen eines Therapieplanes anzuwenden. Das Einsetzen der Medikamentenstripps in die Vorrichtung 3 erfolgt durch die Patientin und ist überaus leicht durchführbar.
Als Träger für den Stoff könnten auch gelartige Formstücke, Vliese od. dgl. vorgesehen werden.

Auf der Außenseite des hermetisch dichten Behälters 1 ist mindestens ein Drucksensor 10 zur Messung des Gewebetonus angebracht. Mit einem derartigen Drucksensor 10 wird indirekt der Behandlungserfolg gemessen. Die Wirkung der Medikamente wird quasi auf dem Gewebetonus meßtechnisch überwacht.

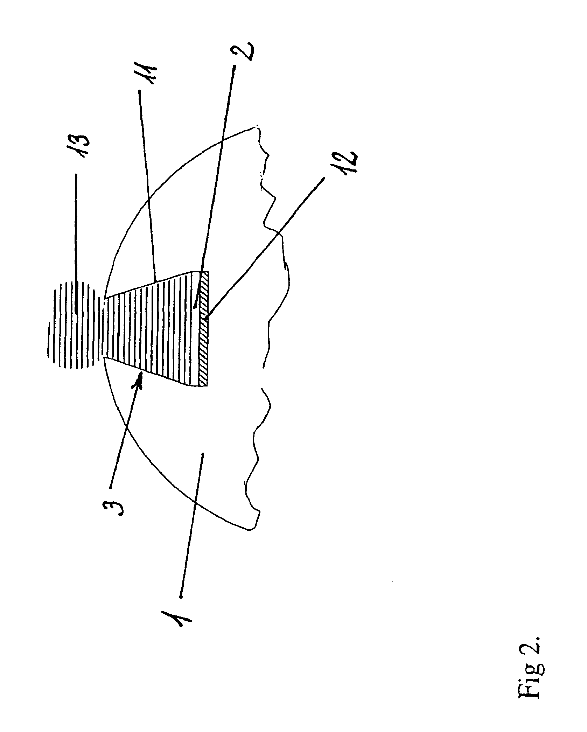

Gemäß der Fig. 2 ist der Bereich der Elektrode 2 und der darüber angeordneten Vorrichtung 3 für die Medikamentenstripps im Gehäuse des Behälters 1 im Detail dargestellt. Das Gehäuse des Behälter 1 weist eine Nut 11, beispielsweise eine Schwalbenschwanznut, auf, in deren Nutgrund eine Wärmefläche 12 angeordnet ist. Über dieser Wärmefläche 12 ist die Elektrode 2 vorgesehen. Über der Elektrode 2 ist ein Stoffträger 13, insbesondere der Medikamemtenstripp, plaziert.

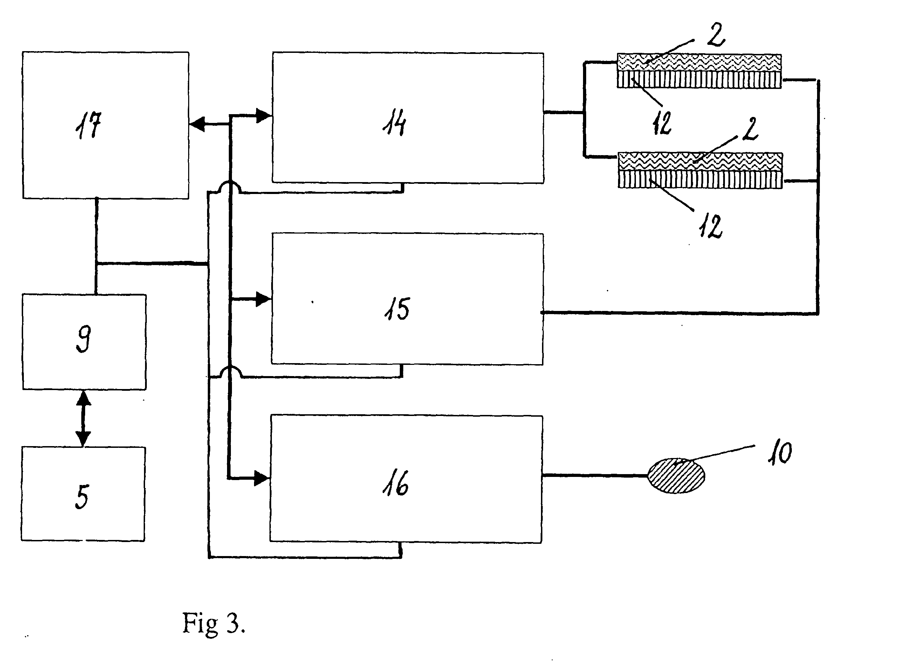

Das in Fig. 1 angesprochene elektronische System 8 der Einrichtung wird an Hand des Prinzipschaltplanes gemäß der Fig. 3 näher erläutert. Das elektronische System 8 umfaßt die Energieversorgung, vorzugsweise einen wiederaufladbaren Akkumulator 9, die über den Berührungsschalter 5 aus- und eingeschaltet wird.
Ferner wird über die Energieversorgung eine programmierbare Stromquelle 14, die mit den Elektroden 2 in Verbindung steht, ein programmierbarer Wärmeregler 15, der mit der Wärmefläche 12 verbunden ist, ein Speicher 16 für die Werte des Drucksensors 10 sowie eine Schnittstelle 17 mit den Schnittstellen-Kontakten 6 mit Energie versorgt.

Über die programmierbare Stromquelle 14 werden die Elektroden 2 und über den programmierbaren Wärmeregler 15 werden die Wärmeflächen 12 definiert geregelt. So kann der Sollwert und die Form des Stromes über die Schnittstelle 17 entsprechend den Einstellungen in digitaler Form vorgegeben werden. Es ist natürlich auch möglich, daß die Stromquelle 14 den eingestellten Sollwert mit dem gemessenen IST-Wert vergleicht und die Differenz ausregelt. Ebenso ist es denkbar und von Vorteil, daß die Dauer des Stromflusses über die Schnittstelle 17 in digitaler Form programmierbar ist. Dabei kann der Strom am Beginn der programmierten Zeit rampenförmig bis zum Erreichen des programmierten Sollwertes ansteigen und am Ende rampenförmig auf Null sinken.

Im Falle der externen Unterbrechung der eingestellten Zeit ist die auf die programmierte Gesamtzeit verbleibende Restzeit abspeicherbar und im Falle des Wiedereinschaltens ist die Restzeit zur Energieabgabe verfügbar. Durch entsprechende Schutzmaßnahmen im Ausgang der Stromquelle 14 ist ein Übersteigen der Leerlaufspannung über einen Grenzwert, beispielsweise 80 V, vermeidbar.

Die programmierbare Stromquelle 14 und der programmierbare Wärmeregler 15 sind also über die Schnittstelle 17, die sowohl kontaktlos wie auch kontaktbehaftet sein kann, und deren Schnittstellen-Kontakte 6 einstellbar. Natürlich ist eine mittels der Schnittstelle 17 programmierbare Wärmeregelung für Temperatur und Zeit zur Ansteuerung der in die Wand des kapselartigen Behälters 1 integrierten elektrischen Wärmefläche 12 realisierbar.

Die Messwerte des Drucksensors 10 sind in einem nichtflüchtigen Speicher 16 aufzeichenbar, wobei die aufgezeichneten Druckmesswerte über die Schnittstelle 17 auslesbar sind.

Ergänzt kann das Vaginal-Iontophoresegerät einerseits durch ein Ladegerät und anderseits durch ein Einstell- bzw. Prüfgerät werden. Das Ladegerät versorgt das eingesetzte Vaginal-lontophoresegerät mit Energie und dient zum Aufladen des integrierten Akkumulators 9. Je nach Stromstärke, Pulsverhältnis und Applikationsdauer ist ein entsprechender Ladevorgang erforderlich. Dies wird beim Einsetzten des gereinigten Vaginal-lontophoresegerät in das Ladegerät optisch angezeigt. Das Einstell- bzw. Prüfgerät, das nur für den Arzt erhältlich ist, erlaubt diesem einerseits die Einstellung von Stromstärke und Pulsverhältnis - in Abhängigkeit von den zu transfundierenden Medikamenten - und den individuellen Bedürfnissen der einzelnen Patienten. Anderseits wird mit diesem Gerät auch die ordnungsgemäße Funktion des Vaginal-Iontophoresegerät und die vorprogrammierten Einstellungen überprüft. Die Patientin selbst kann das Vaginal-lontophoresegerät nur ein - und ausschalten, reinigen, die jeweils verordneten Medikamentenstripps einsetzen und entfernen sowie mittels Ladegerät den Akkumulator 9 aufladen. Eine Veränderung der vom Arzt vorgegebenen lontophoreseparameter ist der Patientin nicht möglich. Für diese Behandlung kommen insbesondere veresterte Sterioide wie Ostriol- Salze, aber auch spezielle, die Durchblutung des Beckenbodens anregende Präparate zum Einsatz. Die Vorteile dieser neuen Einrichtung sind vor allem darin zu sehen, daß eine Behandlung ohne Unterbrechung des normalen Tagesablaufes durchgeführt werden kann, da das Vaginal-Iontophoresegerät intravaginal getragen wird und keinerlei externe Einrichtungen während seines Betriebes benötigt. Je nach Erscheinungsbild der Inkontinenz und Alter der Patientin können verschiedene Wirkstoffkombinationen zum Einsatz kommen, wobei die Medikamentenstripps für Einmalgebrauch fertig vorbereitet und von der Patientin leicht eingesetzt werden können. Bedingt durch das variable Pulsverhältnis in Kombination mit der feinstufig einstellbaren Stromstärke kann für jedes Präparat und für die verschiedenen Grade der Inkontinenzbeschwerden eine optimale Tiefenwirkung zur Durchflutung der Beckenbodenmuskulatur erreicht werden.

Abschließend wird noch ausdrücklich festgehalten, daß der Behälter 1 in Kontakt mit einer Schleimhaut und/oder Haut in einer Körperöffnung, insbesondere im Urogenital- und/oder Vaginal- und/oder Analtrakt und /oder in der Mund- und/oder in der Ohren- und/oder in der Nasenhöhle, angeordnet sein kann.

Der Ordnung halber sei abschließend darauf hingewiesen, daß zum besseren Verständnis des Aufbaus die Teile bzw. deren Bestandteile teilweise unmaßstäblich und/oder vergrößert und/oder verkleinert dargestellt wurden.

## Patentansprüche

1. Einrichtung zum Einbringen von Stoffen, wie Medikamente, die in flüssiger, salbenförmiger oder gelförmiger Konsistenz vorliegen, durch die Haut, durch lontophorese, wobei die Resorption des Stoffes durch ein elektrisches Feld, das durch einen definierten Gleichstrom zwischen zwei Elektroden erzeugbar ist, erfolgt, und ein in Körperöffnungen einführbarer, kapselartiger, hermetisch dichter Behälter vorgesehen ist, der eine Vorrichtung zur Aufnahme des einzubringenden Stoffes und/oder einen mit dem einzubringenden Stoff getränkten oder versehenen Stoffträger aufweist und dieser Behälter in einer Körperöffnung anordenbar ist, so daß er in Kontakt mit einer Schleimhaut und/oder Haut in der Körperöffnung, insbesondere im Urogenital-und/oder Vaginal- und/oder Analtrakt und/oder in der Mund- und/oder in der Ohren- und/oder in der Nasenhöhle, ist, **dadurch gekennzeichnet, daß** der Behälter (1) mindestens zwei Elektroden (2) zum Aufbau des elektrischen Gleichfeldes an seiner Außenseite aufweist und daß im Behälter (1) eine programmierbare Stromquelle (14) vorgesehen ist, die mit den Elektroden (2) in Verbindung steht.

2. Einrichtung nach Anspruch 1, **dadurch gekennzeichnet, daß** durch eine integrierte, elektrisch heizbare Wärmefläche (12) die Oberfläche des kapselartigen Behälters (1) definiert erwärmbar ist.

3. Einrichtung nach Anspruch 1 oder 2, **dadurch gekennzeichnet, daß** die Energieversorgung in Form eines in den hermetisch dichten Behälter (1) integrierten wiederaufladbaren Akkumulators (9) erfolgt.

4. Einrichtung nach Anspruch 1 oder 2, **dadurch gekennzeichnet, daß** die Energieversorgung in induktiver Form von einem externen System erfolgt.

5. Einrichtung nach mindestens einem der Ansprüche 1 bis 4, **dadurch gekennzeichnet, daß** über eine kontaktlose oder kontaktbehaftete Schnittstelle (17) die Parameter der programmierbaren Stromquelle (14) und die Erwärmung einer integrierten Wärmefläche (12) einstellbar sind.

6. Einrichtung nach mindestens einem der Ansprüche 1 bis 5, **dadurch gekennzeichnet, daß** der Sollwert und die Form des Stromes über eine kontaktlose oder kontaktbehaftete Schnittstelle (17) entsprechend den Einstellungen in digitaler Form vorgebbar ist.

7. Einrichtung nach mindestens einem der Ansprüche 1 bis 6, **dadurch gekennzeichnet, daß** die Stromquelle (14) den eingestellten Sollwert mit dem gemessenen IST-Wert vergleicht und die Differenz ausregelt.

8. Einrichtung nach mindestens einem der Ansprüche 1 bis 7, **dadurch gekennzeichnet, daß** durch entsprechende Schutzmaßnahmen im Ausgang der Stromquelle (74) ein Übersteigen der Leerlaufspannung über einen Grenzwert, beispielsweise 80 V, vermeidbar ist.

9. Einrichtung nach mindestens einem der Ansprüche 1 bis 8, **dadurch gekennzeichnet, daß** die Dauer des Stromflusses über eine kontaktlose oder kontaktbehaftete Schnittstelle (17) in digitaler Form programmierbar ist.

10. Einrichtung nach mindestens einem der Ansprüche 1 bis 9, **dadurch gekennzeichnet, daß** der Strom am Beginn der programmierten Zeit rampenförmig bis zum Erreichen des programmierten Sollwertes ansteigt und am Ende rampenförmig auf Null sinkt.

11. Einrichtung nach mindestens einem der Ansprüche 1 bis 10, **dadurch gekennzeichnet, daß** im Falle der externen Unterbrechung der eingestellten Zeit die auf die programmierte Gesamtzeit verbleibende Restzeit abspeicherbar ist und im Falle des Wiedereinschaltens die Restzeit zur Energieabgabe verfügbar ist.

12. Einrichtung nach mindestens einem der Ansprüche 1 bis 11, **dadurch gekennzeichnet, daß** die Stromquelle (14) über einen Berührungsschalter (5) ein- und ausschaltbar ist.

13. Einrichtung nach mindestens einem der Ansprüche 5 bis 12, **dadurch gekennzeichnet, daß** eine mittels der kontaktlosen oder kontaktbehafteten Schnittstelle (17) programmierbare Regelung für Temperatur und Zeit zur Ansteuerung der in die Wand des kapselartigen Behälters (1) integrierten elektrischen Wärmefläche (12) realisierbar ist.

14. Einrichtung nach mindestens einem der Ansprüche 1 bis 13, **dadurch gekennzeichnet, daß** die Vorrichtung (3) zur Aufnahme des einzubringenden Stoffes, insbesondere eines Medikamentes, zum Beispiel offenporige Schaumstoffstripps sind, die mit den anzuwendenden Stoff definiert getränkt und/oder versehen sind.

15. Einrichtung nach mindestens einem der Ansprüche 1 bis 14, **dadurch gekennzeichnet, daß** als Stoffträger (13) gelartige Formstücke vorgesehen sind.

16. Einrichtung nach mindestens einem der Ansprüche 1 bis 13, **dadurch gekennzeichnet, daß** als (13) Stoffträger Vliese od. dgl. vorgesehen sind.

17. Einrichtung nach mindestens einem der Ansprüche 1 bis 16, **dadurch gekennzeichnet, daß** auf der Außenseite des hermetisch dichten Behälters (1) mindestens ein Drucksensor (10) zur Messung des Gewebetonus angebracht ist.

18. Einrichtung nach Anspruch 17, **dadurch gekennzeichnet, daß** die Messwerte des Drucksensors (10) in einem nichtflüchtigen Speicher (16) aufzeichenbar sind.

19. Einrichtung nach Anspruch 18, **dadurch gekennzeichnet, daß** die aufgezeichneten Druckmesswerte über eine kontaktlose oder kontaktbehaftete Schnittstelle (17) auslesbar sind.

## Claims

1. Device for the transdermal administration of substances, such as medicinal products in the form of a liquid, ointment or gel, by means of iontophoresis, whereby the substance is absorbed through an electric field that is produced by a defined direct current between two electrodes, and where a capsule-like, hermetically sealed container that can be introduced into orifices of the body is provided, which has a container for the substance to be administered and/or a substance carrier soaked in or covered with the substance to be administered, and where this container can be inserted into an orifice of the body so that it is in contact with the mucous membrane or skin of this orifice, especially in the urogenital and/or vaginal and/or anal tract and/or the mouth and/or ears and/or nose, **characterised by** the fact that the container (1) has at least two electrodes (2) to produce the electric field on the outside, and that a programmable power source (14) is provided in the container (1), which is connected to the electrodes (2).

2. Device in accordance with Claims 1, **characterised by** the fact that the surface of the capsule-like container (1) can be heated to a defined temperature by means of an integrated, electrical heating unit (12).

3. Device in accordance with Claim 1 or 2, **characterised by** the fact that the power supply is provided in the form of a rechargeable battery (9) integrated in the hermetically sealed container (1).

4. Device in accordance with Claim 1 or 2, **characterised by** the fact that the energy supply is provided inductively by an external system.

5. Device in accordance with at least one of Claims 1 to 4, **characterised by** the fact that the parameters of the programmable power source (14) and temperature of the integrated heating unit (12) can be set via an interface (17) with or without contacts.

6. Device in accordance with at least one of Claims 1 to 5, **characterised by** the fact that the reference value and form of current can be defined digitally in accordance with the settings via an interface (17) with or without contacts.

7. Device in accordance with at least one of Claims 1 to 6, **characterised by** the fact that the power source (14) compares the set reference value with the measured actual value and regulates the difference.

8. Device in accordance with at least one of Claims 1 to 7, **characterised by** the fact that with appropriate protection measures at the output of the power source (14) it is possible to prevent the no-load voltage from exceeding a certain limit, for example 80 V.

9. Device in accordance with at least one of Claims 1 to 8, **characterised by** the fact that the duration of the current can be programmed digitally via an interface (17) with or without contacts.

10. Device in accordance with at least one of Claims 1 to 9, **characterised by** the fact that at the beginning of the programmed time the current increases gradually until the programmed reference value is reached, and then decreases gradually to zero at the end.

11. Device in accordance with at least one of Claims 1 to 10, **characterised by** the fact that the time remaining of the programmed total time can be stored in the case of an external interruption, and the remaining time is available for the release of energy when the power is switched on again.

12. Device in accordance with at least one of Claims 1 to 11, **characterised by** the fact that the power source (14) can be switched on and off with a contact switch (5).

13. Device in accordance with at least one of claims 5 to 12, **characterised by** the fact that a temperature and time control programmable via an interface (17) with or without contacts can be realised to control the electric heating unit (12) integrated in the wall of the capsule-like container (1).

14. Device in accordance with at least one of Claims 1 to 13, **characterised by** the fact that the container (3) for the substance to be administered, in particular a medicinal product, is made for example of open-pored foam strips that are soaked in and/or covered with the defined substance to be administered.

15. Device in accordance with at least one of Claims 1 to 14, **characterised by** the fact that gel-like shaped parts are provided as substance carriers (13).

16. Device in accordance with at least one of Claims 1 to 13, **characterised by** the fact that non-woven fabrics or the like are provided as substance carriers (13).

17. Device in accordance with at least one of Claims 1 to 16, **characterised by** the fact that there is at least one pressure sensor (10) to measure tissue tone on the outside of the hermetically sealed container (1).

18. Device in accordance with Claim 17, **characterised by** the fact that the measurements by the pressure sensor (10) can be recorded in a permanent memory (16).

19. Device in accordance with Claim 18, **characterised by** the fact that the recorded pressure measurements can be read out via an interface (17) with or without contacts.

## Revendications

1. Installation à effectuer pour l' introduction transdermique de substances, comme des médicaments existant sous la forme liquide, de crème ou de gel, au moyen d'une iontophorèse, la substance se résorbant au travers d'un champ électrique, que l' on peut générer à l' aide d'un courant continu défini circulant entre deux électrodes, et un récipient hermétique et sous forme de capsule prévu pour l'introduction dans les orifices du corps, lequel récipient comporte un dispositif pour accueillir la substance à introduire et/ou un support imbibé ou rempli de la substance à introduire, et est configurable dans un orifice corporel, de manière à ce qu' il soit en contact avec une muqueuse et/ou la peau dans l' orifice corporel, notamment par voie urogénitale et/ou vaginale et/ou anale et/ou dans la cavité buccale et/ou auditive et/ou dans la fosse nasale, **se caractérisant par le fait que** le récipient (1) comporte au moins deux électrodes (2) sur sa face externe pour générer le champ électrique en continu et qu'il soit prévu à l'intérieur du récipient (1) une source de courant programmable (14) reliée aux électrodes (2).

2. Installation selon la revendication 1 **caractérisée par le fait que** la surface du récipient sous forme de capsule (1) soit par définition réchauffable au moyen d'une surface calorifique intégrée électriquement chauffable (12).

3. Installation selon la revendication 1 ou 2 **caractérisée par le fait que** l'alimentation en énergie se fasse sous la forme d'un accumulateur rechargeable (9) intégré au récipient hermétiquement étanche (1).

4. Installation selon la revendication 1 ou 2 **caractérisée par le fait que** l'alimentation en énergie se fasse sous la forme inductive d'un système externe.

5. Installation selon au moins une des revendications 1 à 4 **caractérisée par le fait que** les paramètres de la source de courant programmable (14) et la caléfaction d'une surface calorifique soient réglables au moyen d'une interface (17) avec ou sans contact.

6. Installation selon au moins l'une des revendications 1 à 5 **caractérisée par le fait que** la valeur de consigne et la forme du courant soient définissables numériquement au moyen d'une interface avec ou sans contact (17) correspondant aux réglages.

7. Installation selon au moins l'une des revendications 1 à 6 **caractérisée par le fait que** la source de courant (14) compare la valeur de consigne réglée avec la valeur réelle calculée et compense la différence.

8. Installation selon au moins l'une des revendications 1 à 7 **caractérisée par le fait que** l'on puisse éviter un excès du voltage en marche à vide au delà de la valeur limite, 80 V, par exemple, en prenant des mesures de protection à la sortie de la source de courant (14).

9. Installation selon au moins l'une des revendications 1 à 8 **caractérisée par le fait que** la durée du passage du courant soit programmable numériquement au moyen d'une interface avec ou sans contact (17).

10. Installation selon au moins l'une des revendications 1 à 9 **caractérisée par le fait que** le courant augmente progressivement et linéairement au début du temps programmée jusqu'à atteindre la valeur de consigne programmée et diminue progressivement et linéairement jusqu'à zéro à la fin.

11. Installation selon au moins l'une des revendications 1 à 10 **caractérisée par le fait que**, dans le cas d'une interruption externe du temps réglé, le temps restant sur le temps total programmé soit enregistrable et que dans le cas d'un ré-allumage, le temps restant soit disponible pour une remise d'énergie.

12. Installation selon au moins l'une des revendications 1 à 11 **caractérisée par le fait que** la source de courant (14) soit allumable et éteignable grâce à un interrupteur de contact (5).

13. Installation selon au moins l'une des revendications 5 à 12 **caractérisée par le fait que** l'on puisse opérer un réglage programmable de la température et de la durée au moyen d'une interface avec ou sans contact (17) pour adresser la surface calorifique électrique (12) intégrée dans la paroi du récipient (1) sous forme de capsule.

14. Installation selon au moins l'une des revendications 1 à 13 **caractérisée par le fait que** le dispositif (3) de réception de la substance à introduire, notamment d'un médicament, peut être par exemple des bandes de mousse à pores ouvertes, lesquelles bandes de mousse sont par définition imbibées et/ou dotées de la substance à appliquer.

15. Installation selon au moins l'une des revendications 1 à 14, **caractérisée par le fait que** des platines sous forme de gel soient prévues comme supports de la substance (13).

16. Installation selon au moins l'une des revendications 1 à 13, **caractérisée par le fait que** des non-tissés soient prévus comme supports de la substance (13).

17. Installation selon au moins l'une des revendications 1 à 16, **caractérisée par le fait que** au moins un capteur de pression (10) soit apposé sur la face externe du récipient hermétiquement étanche (1) pour mesurer la tonicité du tissu.

18. Installation selon la revendication 17, **caractérisée par le fait que** les valeurs mesurées par le capteur de pression (10) soient enregistrables dans une mémoire fixe (16).

19. Installation selon la revendication 18, **caractérisée par le fait que** les taux de pression mesurés et enregistrés soit lisibles au moyen d'une interface avec ou sans contact.
